Europäisches Patentamt

European Patent Office

Office européen des brevets

(19)

(11) Veröffentlichungsnummer: **0 477 577 A2**

# EUROPÄISCHE PATENTANMELDUNG

(12)

(21) Anmeldenummer: **91114514.2**

(22) Anmeldetag: **29.08.91**

(51) Int. Cl.5: **G01N 27/26**, G01N 27/416, G01N 33/00

(30) Priorität: **27.09.90 DE 4030516**

(43) Veröffentlichungstag der Anmeldung:
**01.04.92 Patentblatt 92/14**

(84) Benannte Vertragsstaaten:
**BE DE FR GB IT NL**

(71) Anmelder: **Drägerwerk Aktiengesellschaft
Moislinger Allee 53-55**

**W-2400 Lübeck 1(DE)**

(72) Erfinder: **Kuhr, Joachim
Altengammer Strasse 14
W-2400 Lübeck(DE)**
Erfinder: **Bahs, Hans-Jürgen, Dr.
Sahlredder 7
W-2401 Offendorf(DE)**

(54) **Verfahren zur Konzenstrationsüberwachung eines gasförmigen Bestandteils in einem abgeschlossenen Raum.**

(57) Ein Verfahren zur Überwachung der Konzentration eines gasförmigen Bestandteils in einem zur Umgebung abgeschlossenen Raum, in welchem eine Atmosphäre vorgegebener Zusammensetzung aufrecht erhalten wird, indem der Istwert der Konzentration von einem Sensor überwacht und bei Erreichen eines Grenzwertes ein entsprechender Anteil des gasförmigen Bestandteils in den Raum hinzudosiert wird, soll derart verbessert werden, daß eine Kalibrierung der Sensoren entfallen kann, oder zumindest in solch langen Zeitabständen erfolgen muß, daß eine Behinderung der Arbeitstätigkeit mit den so ausgestatteten Geräten unerheblich ist.

Dazu ist vorgesehen, daß der Sensor (6) einer Selbstkalibrierung unterworfen wird, indem eine anfängliche Startkonzentration in den Raum (2) dosiert wird, die von dem Sensor (6) als Sollwert an eine Meßwertverarbeitungseinheit (10) weitergeleitet wird. Die Einheit (10) ermittelt daraus einen oberen oder unteren Grenzwert, bei dessen Erreichen bzw. Über- oder Unterschreiten, entweder eine Nachdosierung erfolgt (unterer Grenzwert) oder eine Verdünnung mit Inertgas erfolgt (oberer Grenzwert)

FIG. 1

Die Erfindung betrifft ein Verfahren zur Überwachung der Konzentration eines gasförmigen Bestandteils in einem zur Umgebung abgeschlossenen Raum, in welchem eine Atmosphäre vorgegebener Zusammensetzung aufrecht erhalten wird, indem der Istwert der Konzentration von einem Sensor überwacht und bei Erreichen eines Grenzwertes ein entsprechender Anteil des gasförmigen Bestandteils in den Raum hinzudosiert wird. Außerdem betrifft die Erfindung eine Vorrichtung zur Konzentrationsüberwachung unter Anwendung des genannten Verfahrens.

Derartige Verfahren und Vorrichtungen sind allgemein in der Prozeßtechnik, z.B. in der Chemischen Industrie erforderlich, um bei chemischen Verfahrensreaktionen die für die Reaktion notwendigen Bestandteile stets in gewünschten Konzentrationen vorliegen zu haben. Ein weiterer Anwendungsbereich findet sich in der Desinfektion und Reinigung von kontaminierten Geräten, vor allen Dingen im Bereich der Medizintechnik. Hier sind Sterilisations- und Desinfektionskammern bekannt, welche unter Aufrechterhaltung einer bestimmten Atmosphäre vorgegebener Zusammensetzung über einen vorgeschriebenen Zeitabstand die in den Desinfektionskammern befindlichen Geräte und Instrumente von schädlichen Keimen befreien.

Ganz allgemein ist jedoch das Verfahren unter Verwendung eines jeden Sensors einsetzbar, der die Aufrechterhaltung einer bestimmten Zusammensetzung einer gasförmigen Atmosphäre überwachen oder regeln soll.

In der DE-OS 36 38 789 ist eine Sterilisationskammer angegeben, in der Wasserstoffperoxid ($H_2O_2$) in vorgegebener Menge in einen Verdampfer dosiert wird, durch den innerhalb der Sterilisationskammer eine gasförmige Atmosphäre mit einer vorgegebenen Konzentration an $H_2O_2$ hergestellt wird. Zur Überwachung der Innenatmosphäre der Kammer ist ein mit dem Sterilisationsmittel (Wasserstoffperoxid) reagierender Sensor vorgesehen, der mittels einer Steuereinheit dafür sorgt, daß bei sich verändernder Zusammensetzung der Desinfektionsatmosphäre eine entsprechende Zudosierung von Wasserstoffperoxid erfolgt. Als Beispiel eines auf Wasserstoffperoxid reagierenden Sensors ist die Kombination zweier Temperaturfühler angegeben, von denen der eine in der Lage ist, mit dem Wasserstoffperoxid zu reagieren, und der andere vor dem Zutritt von Wasserstoffperoxid abgeschirmt ist. Durch entsprechende Vergleichsmessung erzeugt der Sensor ein Ausgangssignal, welches für die jeweilige Menge des Konzentrats innerhalb der Atmosphäre repräsentativ ist. Neben den genannten temperaturempfindlichen Sensoren gibt es auch elektrochemische Meßzellen. Bei Auswahl eines geeigneten Elektrolyts mit zugehöriger Meß- und Gegenelektrode, an die eine Arbeitsspannung angelegt wird, kann ein auf das Desinfektionsmittel ansprechendes Meßsignal gewonnen werden. Hierzu gehören beispielsweise elektrochemische Sensoren zum Nachweis von Formaldehyd, wenn als Desinfektionsmittel Formaldehyddampf verwendet wird. Derartige Sensoren besitzen Meß- und Gegenelektroden aus Platin, die in einem Elektrolyten aus verdünnter Schwefelsäure getaucht sind.

Die bekannten Sensoren zur Anwendung in der genannten Vorrichtung haben den Nachteil, daß durch ihre Reaktion mit den vorhandenen Desinfektionsmitteln ihre Eigenschaften sich nachhaltig und irreversibel ändern, was Einfluß auf ihre Empfindlichkeit und Signalgröße hat. So ist zum Beispiel der dem Wasserstoffperoxid ausgesetzte temperaturempfindliche Sensor als Platinwiderstands-Temperaturdetektor ausgebildet, wobei wegen des aktiven Oxidationsmittels Wasserstoffperoxid der Platindraht angegriffen wird und sich in seinen physikalischen Eigenschaften als Widerstandselement verändert. Elektrochemische Sensoren zum Nachweis von Formaldehyd zeigen im Laufe der Lebensdauer Drifteigenschaften und Empfindlichkeitsveränderungen, welche von Veränderungen im Elektrolyten und an den Oberflächen vor allem der Meßelektrode herrühren. Als Material für die Meßelektroden wird ebenfalls Platin verwendet. Derartige Drifteigenschaften und Veränderungen der Empfindlichkeit machen ein wiederholtes Kalibrieren der Sensoren mit einem Kalibriergas notwendig, so daß der Betrieb von z.B. Desinfektionskammern durch oftmalige Kalibrierung der Sensoren gestört ist bzw. die Desinfektionszyklen in unerwünschter Weise über einen längeren Zeitraum unterbrochen werden müssen. Außerdem ist zum Zwecke der Kalibrierung stets ein Kalibriergas von vorgegebener Zusammensetzung bereitzuhalten.

Der vorliegenden Erfindung liegt somit die Aufgabe zugrunde, ein Verfahren der genannten Art so zu verbessern, daß eine Kalibrierung der Sensoren wegfallen kann, oder zumindest nur in solch langen Zeitabständen erfolgen muß, daß eine Behinderung der Arbeitstätigkeit mit den so ausgestatteten Geräten unerheblich ist.

Die Lösung der Aufgabe erfolgt dadurch, daß zur Einstellung der Konzentration im Bereich des gewünschten Sollwertes in den Raum eine anfängliche Startmenge des gasförmigen Bestandteils dosiert wird, die von dem Sensor als Startkonzentrationswert registriert wird, welcher von einer Meßwertverarbeitungseinheit aufgenommen und um einen Bruchteil seines Wertes verändert wird, und daß der veränderte Wert als Grenzwert gespeichert wird, bei dessen Erreichen durch den Konzentrations-Istwert eine zur Erlangung des Sollwertes erforderliche Nachdosierung des gasförmigen Bestandteils erfolgt, falls der Grenzwert unterhalb des Sollwertes liegt, oder eine Inertgasspülung erfolgt, falls der Grenzwert oberhalb des Sollwertes liegt. Als Inertgas wird in diesem Zusammenhang jedes Gas angesehen, das frei von dem zu überwachenden gasförmigen Bestandteil ist. Insbesondere kann dies die Umgebungsluft sein, oder es kann eine gesondert aufbewahrte Gasreserve sein, z.B. in Druckgasflaschen, welche in ihrer speziellen Zusammensetzung die Desinfektion oder den zu regelnden Prozeß nicht beeinflußt.

Der Vorteil der Erfindung liegt im wesentlichen darin, daß der Sensor zur Kontrolle und Regelung nicht mehr die absolute Konzentrationsmenge messen muß, sondern die von dem Gerät vorgegebene, vorher berechnete notwendigerweise zu dosierende Menge an gasförmigem Bestandteil innerhalb des abgeschlossenen Raumes wird von dem Sensor als vorgegebener Startwert registriert, auf den sich der oder die Grenzwerte beziehen, welche von der Meßwertverarbeitungseinheit ermittelt werden. Somit ist die in dem abgeschlossenen Raum sich einstellende Konzentration zu Beginn der Messung als Referenzwert für den Sensor anzusehen, der gleichzeitig der Festlegung der Grenzwerte dient. Veränderungen der Sensoreigenschaften, wie zum Beispiel Driften oder Veränderung der Empfindlichkeit, spielen bei der Festlegung der Grenzwerte keine Rolle. Der erforderliche Sensor zur Regelung der Sollwertkonzentration wird durch dieses Verfahren automatisch vor Beginn des Regelungsprozesses kalibriert, indem er den anfänglichen Startkonzentrationswert als Referenzwert ansieht. Auf diese Weise können mehrere Prozeßzyklen, z.B. im Zuge einer Desinfektion oder Sterilisation von medizintechnischen Geräten, hintereinander durchgeführt werden, ohne daß der Sensor zwischendurch kalibriert werden muß. Veränderungen seiner Empfindlichkeit oder ein Driftverhalten wird bei der Selbstkalibrierung während der anfänglichen Erzeugung des Startkonzentrationswertes in dem fraglichen abgeschlossenen Raum berücksichtigt. Die Festlegung der Grenzwerte kann entweder so erfolgen, daß ein unterer Grenzwert unterhalb des Sollwertes ermittelt wird, um zu verhindern, daß der Konzentrationswert unerlaubterweise absinkt, oder es wird ein Grenzwert oberhalb des Sollwertes ermittelt, um zu verhindern, daß der Konzentrationswert unerlaubterweise ansteigt, etwa infolge einer Fehldosierung, oder einer anfänglichen zu hoch eingestellten Startdosierung. Alternativ können auch oberer und unterer Grenzwert gleichzeitig ermittelt werden und zur Überwachung dienen.

Wenn sich nun im Zuge des Desinfektions- oder Sterilisationsprozesses die Konzentration des gasförmigen Bestandteils in der abgeschlossenen Kammer soweit verringert, daß der untere Grenzwert erreicht bzw. unterschritten wird, wird durch Aktivierung einer Dosiervorrichtung eine zusätzliche Menge des die Desinfektion bewirkenden gasförmigen Bestandteils in den Raum dosiert. Diese Zu- bzw. Nachdosierung erfolgt sooft, wie der Konzentrations-Istwert während des erforderlichen Desinfektionszeitraumes den unteren Grenzwert erreicht bzw. unterschreitet. Dabei wird der untere Grenzwert so gewählt, daß eine wirksame Desinfektion bzw. Sterilisation mit einem entsprechenden Konzentrations-Istwert sichergestellt bleibt. Sollte während einer Nachdosierung der Konzentrations-Istwert über einen oberen Grenzwert hinaus erreicht werden, wird zu dessen Herabsetzung eine Spülung mit inertem Gas durchgeführt, wodurch der Bestandteil des die Desinfektion bewirkenden Gases verringert wird. Dies kann dann erforderlich sein, wenn es durch eine erhöhte Konzentration zu Schädigungen des im abgeschlossenen Raum befindlichen Desinfektionsgutes kommen könnte. In der Regel aller Fälle reicht es jedoch aus, nur einen unteren Grenzwert festzulegen, um die desinfektionswirksame Konzentration aufrecht zu erhalten. Sowohl bei der Nachdosierung als auch bei der Inertgasspülung wird das in der Kammer verdrängte Gasvolumen über eine Abluftleitung entfernt.

Wenn im vorangehenden und im folgenden der Desinfektions- bzw. Sterilisationsprozeß beispielhaft zur Erläuterung herangezogen ist, ist es im Umfange der Erfindung ebenso angebracht, das Verfahren für andere Prozesse einzusetzen, wie sie z.B. zum Aufrechterhalten einer bestimmten Atmosphäre in Speicherräumen zur Konservierung von Lebensmitteln, Obst bzw. Gemüse oder Fleisch, erforderlich sind.

Es kann durch übermäßiges Einstellen eines Startkonzentrationswertes oder durch zu häufiges Nachdosieren, aber auch durch fehlerhafte Funktion der Dosier- bzw. Spülvorrichtung, der obere bzw. untere Grenzwert soweit über- bzw. unterschritten werden, daß eine wirksame Desinfektion nicht mehr sichergestellt oder daß eine fehlerfreie Gerätefunktion nicht mehr gewährleistet ist. Um diesen Fehlern Rechnung zu tragen, ist es zweckmäßig, oberhalb des oberen bzw. unterhalb des unteren Grenzwertes durch die Meßwertverarbeitungseinheit jeweils einen weiteren oberen bzw. unteren Sicherheitsgrenzwert festzulegen. Bei Erreichen bzw. Über- oder Unterschreiten der jeweiligen Sicherheitsgrenzwerte wird eine Warnvorrichtung betätigt, die den Anwender über einen fehlerhaften Zustand informiert. Es ist ebenso möglich, daß die Warnvorrichtung jedesmal dann betätigt wird, wenn der Konzentrations-Istwert ein Sicherheitsband erreicht, das durch die Lage des oberen bzw. unteren Grenzwertes und des dazugehörigen Sicherheitsgrenzwertes bestimmt ist.

In weiterer Ausgestaltung der Erfindung ist es zweckmäßig, die korrekte Funktionsweise der Dosiervorrichtung zu überprüfen. Dazu wird die Dosiervorrichtung in der weise angesteuert, daß sie eine mehrfache bzw. bruchteilige Menge des gasförmigen Bestandteils in den Raum der Kammer dosiert, als zur Erreichung des Sollwertes notwendig ist. Eine korrekte Dosierung ist dann erfolgt, wenn der vom Sensor gemessene Konzentrations-Istwert einen oberhalb bzw. unterhalb des Sollwertes befindlichen, vorgebbaren Kontrollwert erreicht oder überschreitet. Der Kontrollwert kann auch mit einem der beiden Werte: oberer Grenzwert oder oberer Sicherheitsgrenzwert zusammenfallen; er kann entweder von Hand über eine Eingabeeinheit in die Meßwertverarbeitungseinheit eingegeben oder von ihr durch eine festgelegte Rechenoperation, analog zu den übrigen Grenzwerten, mit Hilfe des Startkonzentrationswertes ermittelt werden. Die Durchführung einer derartigen Kontroll-Dosierung kann auf Anforderung durch den Anwender mit Hilfe entsprechender Schalter- oder Tastenbetätigung an der Eingabevorrichtung der Meßwertverarbeitungseinheit durchgeführt werden, oder die Meßwertverarbeitungseinheit ist derart programmiert, daß sie über eine Freigabeleitung nach einer vorgebbaren Anzahl von Desinfektionszyklen einen Kontrollzyklus für die Dosiervorrichtung durchläuft. Es wird dann beispielsweise die doppelte bzw. die halbe Menge an Desinfektionsmittel dosiert und der Verlauf des Konzentrations-Istwertes registriert.

Um sicherzugehen, daß der erreichte Kontrollwert nicht über die Maßen überschritten wird, was durch eine fehlerhafte Überdosierung erfolgen könnte, wird ein oberhalb des Kontrollwertes liegender Überwachungswert vorgegeben. Der Konzentrations-Istwert hat dann bei korrekter Dosierung innerhalb des Meßwert-Bandes von Kontrollwert und Überwachungswert zu liegen.

Ein vereinfachtes und für die meisten Anwendungszwecke ausreichendes Verfahren besteht darin, daß der Startkonzentrationswert auf 110% des Sollwertes festgelegt wird und daß der Bruchteil so bemessen ist, daß als Grenzwert 100% des Sollwertes gespeichert wird. Auf diese Weise stellt man sicher, daß während des gesamten Desinfektionszyklus 100% des Sollwertes aufrechterhalten bleiben, und bei Erreichen des Sollwertes die Konzentration kurzzeitig um 10% erhöht wird. Somit erreicht man eine periodische Schwankung des Konzentrations-Istwertes innerhalb des abgeschlossenen Raumes oberhalb des Sollwertes. Eine andere Möglichkeit besteht darin, den Startkonzentrationswert ebenfalls auf 110% des Sollwertes festzulegen, den Bruchteil jedoch so zu bemessen, daß als Grenzwert 90% des Sollwertes gespeichert wird. Auf diese Weise schwankt der Konzentrations-Istwert innerhalb der Kammer um +/- 10% um den Sollwert herum. Die Wahl anderer Startkonzentrationswerte und Grenzwerte ermöglichen eine Variation der Schwankungsbreiten um den Sollwert herum, die je nach Erfordernis an den entsprechenden Anwendungsfall angepaßt werden können.

Eine Vorrichtung zur Überwachung der Konzentration eines gasförmigen Bestandteils in einem zur Umgebung abgeschlossenen Raum unter Anwendung des Verfahrens nach Anspruch 1 ist dadurch gekennzeichnet, daß die Signalleitung des Sensors an die Meßwertverarbeitungseinheit angeschlossen ist, die den erreichten Startkonzentrationswert aufnimmt und den errechneten Grenzwert in einem Vergleicher ablegt, der gleichzeitig an die Signalleitung des Sensors zum Vergleich des Grenzwertes mit dem Konzentrations-Istwert angeschlossen ist. Der Vergleicher steuert eine Dosiervorrichtung derart an, daß eine zur Wiedererlangung des Startkonzentrationswertes abgemessene Menge des gasförmigen Bestandteiles in den Raum hinzudosiert wird, sobald der Konzentrations-Istwert einen Grenzwert unterhalb des Sollwertes erreicht oder unterschreitet. Die Vorrichtung sorgt dafür, daß der eingestellte Grenzwert während des gesamten Desinfektionszyklus innerhalb der Regeltoleranzen aufrechterhalten wird.

Für den Fall, daß eine übermäßige Zudosierung des gasförmigen Bestandteils erfolgt ist, wird inertes Spülgas in den Raum dosiert, sobald der Konzentrations-Istwert einen Grenzwert oberhalb des Sollwertes erreicht bzw. überschreitet, wodurch die Konzentration des gasförmigen Bestandteiles verdünnt wird. Eine Inertgasspülung ist besonders dann zweckmäßig, wenn zur Aufrechterhaltung der Desinfektion nicht nur ein unterer Grenzwert eingehalten werden muß, sondern wenn das Desinfektionsgut eine übermäßige Konzentration des die Desinfektion bewirkenden gasförmigen Bestandteils nicht ausgesetzt werden darf.

Zweckmäßigerweise ist zwischen dem Speicher und dem Vergleicher eine Überwachungseinheit vorgesehen, die den Vergleicher erst dann zur Abgabe der Steuersignale aktiviert, wenn in der Meßwertverarbeitungseinheit nach Ermittlung des Grenzwertes ein Freigabesignal über eine Freigabeleitung an dem Vergleicher ansteht. Wenn normalerweise der Vergleicher ein Steuersignal abgibt, sobald die Übereinstimmung zwischen Konzentrations-Istwert und berechnetem Grenzwert vorhanden ist, kann es zweckmäßig sein, die Ansteuerung entweder der Dosiervorrichtung oder der Spüleinrichtung solange auszusetzen, bis weitere schaltungstechnische oder verfahrensbedingte Maßnahmen erfolgt sind. Wenn z.B. ein gasförmiger Bestandteil erst durch Verdampfen einer Flüssigkeit erzeugt werden muß, bevor er in den abgeschlossenen Desinfektionsraum dosiert wird, muß zunächst sichergestellt werden, daß die Verdampfung der erforderlichen Menge Flüssigkeit erfolgt ist, so daß die Dosierung erst danach stattfinden kann, obwohl der Konzentrations-Istwert den Grenzwert schon erreicht hat.

Die korrekte Dosierfunktion kann auch dadurch erkannt werden, daß der Konzentrations-Istwert unterhalb eines vorgebbaren Kontrollwertes bleibt. Bei Durchführung einer Kontrolldosierung sind die übrigen Alarm- oder Überwachungsfunktionen stillgesetzt.

Es zeigen

Fig. 1: Ein Blockschaltbild einer Desinfektionskammer,

Fig. 2: Ein Zeitablaufschema für das Desinfektionsverfahren mit der Kammer nach Fig. 1.

Ein Ausführungsbeispiel der Erfindung wird anhand einer schematischen Zeichnung dargestellt und im folgenden näher erläutert.

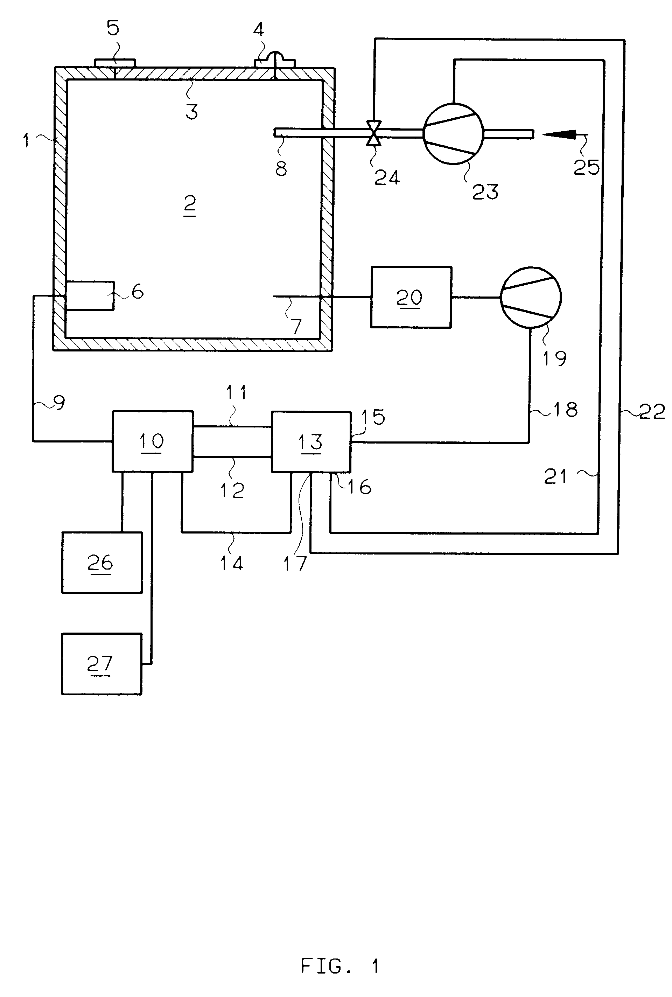

In der Figur 1 ist ein Blockschema einer Vorichtung zur Durchführung einer Desinfektion dargestellt. Eine Desinfektionskammer (1) umschließt einen von der Umgebung abgeschlossenen Raum (2), in den über eine Tür (3) das zu desinfizierende Gut eingebracht werden kann. Die Tür (3) ist über Scharniere (4) und eine Verriegelung (5) betätigbar. Innerhalb des Raumes (2) befindet sich ein Sensor (6), welcher beispielhaft als ein elektrochemischer Sensor ausgebildet ist. Weiterhin ragen in den Raum (2) sowohl eine Dosierleitung (7) als auch ein Spülkanal (8). Der Sensor (6) ist über eine Sensorleitung (9) an eine Meßwertverarbeitungseinheit (10) angeschlossen, welche auch ein Rechenwerk zur Ermittlung eines Grenzwertes aus dem Meßwert des Sensors (6) beinhaltet. In der Meßwertverarbeitungseinheit (10) wird auch der Startkonzentrationswert zu Beginn der Desinfektion aufgenommen, wie er von dem Sensor (6) über die Sensorleitung (9) zugeführt wird. Die Meßwertverarbeitungseinheit (10) ist über eine Übertragungsleitung (11) und eine Grenzwertleitung (12) mit einem Vergleicher (13) verbunden. Die Übertragungsleitung (11) übermittelt dem Vergleicher (13) die aktuellen Konzentrations-Istwerte, wie sie vom Sensor (6) der Meßwertverarbeitungseinheit (10) zugeleitet werden. Über die Grenzwertleitung (12) erhält der Vergleicher (13) sein zweites Vergleichssignal. Eine direkte Verbindung zwischen der Meßwertverarbeitungseinheit (10) und dem Vergleicher (13) bildet eine Freigabeleitung (14). An dieser Freigabeleitung (14) wird dem Vergleicher (13) das Freigabesignal zugeführt, welches eine Priorität gegenüber den Signalen aus der Übertragungsleitung (11) und der Grenzwertleitung (12) besitzt. Erst wenn an allen Leitungen (11, 12, 14) ein Signal ansteht, werden die Ausgänge (15, 16, 17) des Vergleichers (13) aktiviert. Über dieselbe Freigabeleitung (14) kann von der Meßwertverarbeitungseinheit (10) ein Signal zur Durchführung eine Kontrolldosierung an den Vergleicher (13) abgegeben werden. Wenn dies der Fall ist, hat dieses Kontrollsignal Priorität über alle anderen, und die Kontrolldosierung wird durchgeführt ungeachtet des Signalzustandes an den übrigen Leitungen (11,12). Der eine Ausgang (15) des Vergleichers (13) ist über eine Dosierleitung (18) an eine Dosierpumpe (19) angeschlossen, welche bei entsprechender Aktivierung aus einem Vorratsbehälter (20) den die Desinfektion bewirkenden Gasbestandteil über den Dosierkanal (7) in den Innenraum (2) der Desinfektionskammer (1) einbringt. Sobald die Konzentration des gasförmigen Bestandteiles in der Desinfektionskammer (1) angestiegen ist, wird der Vergleicher (13) desaktiviert, da an der Übertragungsleitung (11) und der Grenzwertleitung (12) unterschiedliche Signale anstehen. Bei Aktivierung der Ausgänge (16) und (17) am Vergleicher (13) wird über eine Spülleitung (21) und eine Ventilleitung (22) eine Spülpumpe (23) sowie ein Spülventil (24) betätigt. Über eine Eingabeeinheit (26) können durch den Anwender obere und/ oder untere Sicherheitsgrenzwerte oder ein Kontrollwert eingegeben werden, wenn diese nicht durch die Meßwertverarbeitungseinheit (10) ermittelt werden sollen. Auch die Durchführung einer Kontrolldosierung kann an der Eingabeeinheit (26) ausgelöst werden. Ein typischer Verfahrensablauf kann folgendermaßen stattfinden: auf Knopfdruck durch den Bediener an der Eingabeeinheit (26) wird der Dosierpumpe (19) während einer vorgegebenen Zeit über die Dosierleitung (18) ein Signal zugeführt, durch welches eine bestimmte Menge des die Desinfektion bewirkenden gasförmigen Bestandteils über den Dosierkanal (7) in den Raum (2) der Desinfektionskammer (1) eingeleitet wird. Daraufhin stellt sich ein Anfangskonzentrationswert im Raum (2) ein, der vom Sensor (6) gemessen und der Meßwertverarbeitungseinheit (10) zugeführt wird. Daraus wird in der Meßwertverarbeitungseinheit (10) ein unterer Grenzwert ermittelt und über die Grenzwertleitung (12) dem Vergleicher (13) zugeführt, welcher gleichzeitig über die Übertragungsleitung (11) den aktuellen Konzentrations-Istwert des Sensors (6) empfängt. Wenn dieser Wert an der Übertragungsleitung (11) dem unteren Grenzwert an der Grenzwertleitung (12) entspricht, und der Vergleicher (13) über die Freigabeleitung (14) ein Freigabesignal empfangen hat, wird am Ausgang (15) des Vergleichers (13) über die Dosierleitung (18) ein Signal an die Dosierpumpe (19) gegeben, welches aus dem Vorratsbehälter (20) die erforderliche Menge an gasförmigen Bestandteilen des Desinfektionsmittels in den Raum (2) dosiert, um den auf den Grenzwert abgefallenen Konzentrations-Istwert auf den anfänglichen Sollwert anzuheben. Liegt hingegen der Grenzwert an der Grenzwertleitung (12) oberhalb des Sollwertes, wird, sobald der Konzentrations-Istwert diesen oberen Grenzwert erreicht oder überschreitet, am Ausgang (16) und (17) des Vergleichers (13) über die Spülleitung (21) und die Ventilleitung (22) jeweils ein Signal an die Spülpumpe (23) und das Spülventil (24) gegeben, wodurch aus der Umgebung in Richtung des Strömungspfeils (25) als Inertgas über den Spülkanal (8) in den Raum (2) der Desinfektionskammer (1) geleitet wird. Dadurch wird der gasförmige Bestandteil an Desinfektionsmittel um einen solchen Betrag verdünnt, daß sein aktueller Meßwert wieder auf den gewünschten Sollwert abgesenkt wird.

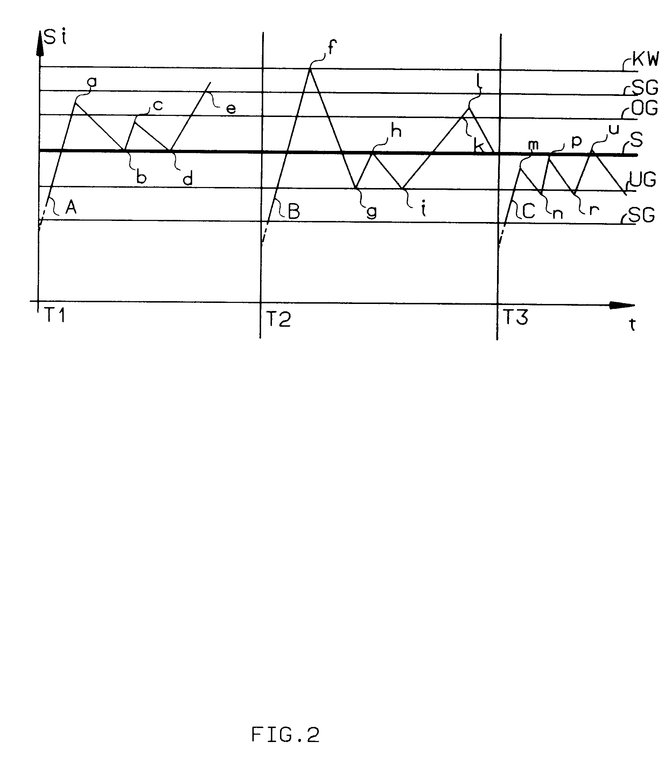

In Figur 2 sind in abgekürzter Zeitreihenfolge drei verschiedene Desinfektionszyklen dargestellt, und zwar zu gewissen Zeitpunkten $T_1$, $T_2$ und $T_3$ an der horizontalen Zeitachse t beginnend, wobei der tatsächliche Beginn der Zyklen schon früher erfolgt ist, dieser frühere Teil jedoch nicht dargestellt ist.

Die Darstellungen beginnen jeweils mit der Dosierung der an der Eingabeeinheit (26) vorgegebenen Startkonzentration A, B, C. Die Konzentration A ist an dem Punkt a erreicht, und der dazugehörige Startkonzentrationswert wird vom Sensor (6) aufgenommen und ein entsprechendes Sensorsignal Si-(a) an die Meßwertverarbeitungseinheit (10) abgegeben. Aus dem Startkonzentrationswert, der bei etwa 110%. des Sollwertes S liegt, wird der Sollwert S sowie der untere (UG) und der obere Grenzwert (OG) und der Sicherheitsgrenzwert (SG) durch die Verarbeitungseinheit (10) ermittelt.

Wenn der Startkonzentrationswert (A) in Punkt (a) erreicht ist, hört die Dosierung auf, und im Laufe der Zeit (Einwirkungszeit genannt) fällt der Konzentations-Istwert auf den Sollwert (s) längs des Kurvenabschnittes (a-b) ab. Das Sensorsignal Si(b) gibt an den Vergleicher (13) die Information, die er benötigt, um die Dosiervorrichtung (19,20) anzusteuern. Die daraufhin ausgelöste Nachdosierung läßt den Konzentrations-Istwert bis zum Punkt (c) ansteigen, bleibt jedoch unterhalb des oberen Grenzwertes (OG). Während der Einwirkzeit nimmt der Konzentrations-Istwert einen Verlauf längs der Strecke (c-d) an. Dieser Arbeitszyklus (Folge von Dosierphase und Einwirkzeit) kann sich mehrmals wiederholen, jenachdem wie die Meßwertverarbeitungseinheit (10) zur Auslösung einer Anzahl von Dosier- bzw. Inertgasspülphasen programmiert ist. Bei dem Desinfektionszyklus $T_1$ folgt dem letzten Arbeitszyklus ein Anstieg des Konzentrations-Istwertes bis zum Sicherheitsgrenzwert SG in Punkt (e). Das dazugehörige Sensorsignal Si(e) löst eine Alarmmeldung an der Warneinrichtung (27) aus. In diesem Falle hat beispielsweise die Dosiervorrichtung (19,20) zuviel dosiert, und die Spülvorrichtung (23,24) hat nicht angesprochen.

Ein nächster Desinfektionszyklus beginnt zum Zeitpunkt $T_2$. Dabei wird zuvor eine Kontrolle der Dosierung durchgeführt: Durch gezielte Mehrfachdosierung bzw. Überdosierung steigt der Konzentrations-Istwert über den Startkonzentrationswert (B)an, bis er in die Nähe des Kontrollwertes (KW) kommt, ihn evtl. auch erreicht oder sogar überschreitet. Dadurch wird eine korrekte Funktion der Dosierung erkannt, und der Konzentrations-Istwert fällt während der Einwirkzeit (f-g) auf den unteren Grenzwert (UG) ab. Dieser beträgt 90% des Sollwertes (S), welcher als Startkonzentrationswert für diesen Desinfektionszyklus vorgegeben war. Die anschließende Dosierphase läßt die Konzentration von (g) nach (h) ansteigen, bis sie den Sollwert (S) erreicht hat. Die nachfolgende Dosierphase, beginnend in Punkt (i), läßt den Konzentrations-Istwert ungewollt deutlich über den Sollwert (S) ansteigen, bis er in Punkt (k) den oberen Grenzwert (OG) schneidet. Durch die Meßwertverarbeitungseinheit (10) und den Vergleicher (13) wird die Spülvorrichtung (23,24) in Tätigkeit gesetzt, worauf Inertgas aus der Umgebung in die Kammer (1) gefördert wird, so daß der Konzentrations-Istwert von Punkt (l) an bis auf den Sollwert (S) absinkt. Bei Erreichen des oberen Grenzwertes (OG) in Punkt (k) kann ebenfalls eine Alarmmeldung an der Warnvorrichtung (27) ausgelöst werden.

Bei einem weiteren beispielhaften Dosierzyklus C ist der Startkonzentrationswert mit 100% des Sollwertes (S) vorgegeben. Der anfängliche Konzentrations-Istwert wird in Punkt (m) erreicht. Die nachfolgende Einwirkzeit löst in Punkt (n) eine Nachdosierung aus, die den Konzentrations-Istwert bis (p) ansteigen läßt. Die nächste Dosierphase läßt den Istwert bis auf (n) leicht über den Sollwert (S) ansteigen. Hier ist also ein Beispiel gezeigt, wie mit anfänglicher Startdosierung und späteren Nachdosierungen ein schnelles Erreichen und Erhalten des Sollwertes (S) verwirklicht wird, ohne daß während der Startdosierung eine übermäßige Konzentration eingestellt wird. Dies kann insbesondere dann erwünscht sein, wenn sich empfindliches Desinfektionsgut in der Kammer (1) befindet, das vor hohen Konzentrationen geschützt bleiben muß.

## Patentansprüche

1. Verfahren zur Überwachung der Konzentration eines gasförmigen Bestandteils in einem zur Umgebung abgeschlossenen Raum, in welchem eine Atmosphäre vorgegebener Zusammensetzung aufrecht erhalten wird, indem der Istwert der Konzentration von einem Sensor überwacht und bei Erreichen eines Grenzwertes ein entsprechender Anteil des gasförmigen Bestandteils in den Raum hinzudosiert wird, dadurch gekennzeichnet, daß zur Einstellung der Konzentration im Bereich des gewünschten Sollwertes in den Raum (2) eine anfängliche Startmenge des gasförmigen Bestandteils dosiert wird, die von dem Sensor (6) als Startkonzentrationswert registriert wird, welcher von einer Meßwertverarbeitungseinheit (10) aufgenommen und um einen Bruchteil seines Wertes verändert wird, und daß der veränderte Wert als Grenzwert gespeichert wird, bei dessen Erreichen durch den vom Sensor (6) gemessenen Konzentrations-Istwert eine zur Erlangung des Sollwertes erforderliche Nachdosierung des gasförmigen Bestandteils erfolgt, falls der Grenzwert unterhalb des Sollwertes liegt, oder eine Spülung mit Inertgas erfolgt, falls der Grenzwert oberhalb des Sollwertes liegt.

2. Verfahren nach Anspruch 1, dadurch gekennzeichnet, daß die Meßwertverarbeitungseinheit (10) einen weiteren oberen und/oder unteren Sicherheitsgrenzwert berechnet, die oberhalb bzw. unterhalb des oberen bzw. unteren Grenzwertes liegen, und daß eine Warneinrichtung (27) betätigt wird, sobald der Konzentrations-Istwert des Sensors (6) den oberen und/oder unteren Sicherheitsgrenzwert erreicht bzw. über- oder unterschreitet.

3. Verfahren nach Anspruch 1 oder 2, dadurch gekennzeichnet, daß zur Überprüfung der Dosierung die Dosiervorrichtung (19,20) eine mehrfache bzw. bruchteilige als zur Erlangung des Sollwertes notwendige Menge des gasförmigen Bestandteils in den Raum (2) dosiert, und daß eine korrekte Dosierung dadurch erkennbar ist, daß der Konzentrations-Istwert einen oberhalb bzw. unterhalb des Sollwertes befindlichen, vorgebbaren Kontrollwert erreicht oder überschreitet.

4. Verfahren nach Anspruch 3, dadurch gekennzeichnet, daß zur Überprüfung der Dosierung die Dosiervorrichtung (19,20) eine mehrfache als zur Erlangung des Sollwertes notwendige Menge des gasförmigen Bestandteils in den Raum (2) dosiert, und daß eine korrekte Dosierung dadurch erkennbar ist, daß der Konzentrations-Istwert unterhalb eines weiteren vorgebbaren Überwachungswertes jedoch oberhalb des Kontrollwertes bleibt.

5. Verfahren nach einem der Ansprüche 1 bis 4, dadurch gekennzeichnet, daß als Startkonzentrationswert 110% des Sollwertes festgelegt wird, und daß der Bruchteil so bemessen ist, daß als Grenzwert 100% des Sollwertes gespeichert wird.

6. Vorrichtung zur Überwachung der Konzentration eines gasförmigen Bestandteils in einem zur Umgebung abgeschlossenen Raum unter Anwendung des Verfahrens nach Anspruch 1, dadurch gekennzeichnet, daß die Signalleitung (9) des Sensors (6) an die Meßwertverarbeitungseinheit (10) angeschlossen ist, die den erreichten Startkonzentrationswert aufnimmt und den errechneten Grenzwert in einem Vergleicher (13) ablegt, der gleichzeitig an die Signalleitung (9) des Sensors (6) zum Vergleich des Grenzwertes mit dem vom Sensor (6) gemessenen Konzentrations-Istwert angeschlossen ist, und welcher eine Dosiervorrichtung (19, 20) derartansteuert, daß sie bei Erreichen des Konzentrations-Istwertes eines unterhalb des Sollwertes liegenden Grenzwertes eine zur Wiedererlangung des Sollwertes abgemessene Menge des gasförmigen Bestandteiles in den Raum (2) dosiert.

7. Vorrichtung nach Anspruch 6, dadurch gekennzeichnet, daß der Vergleicher (13) eine Spüleinrichtung (23, 24) derart ansteuert, daß sie eine zur Wiedererlangung des Sollwertes erforderliche abgemessene Menge von Inertgas in den Raum (2) dosiert, sobald der Konzentrations-Istwert einen oberhalb des Sollwertes liegenden Grenzwert erreicht oder überschreitet.

8. Vorrichtung nach einem der Ansprüche 6 und 7, dadurch gekennzeichnet, daß die Meßwertverarbeitungseinheit (10) eine Überwachungseinheit besitzt, die den Vergleicher (13) erst dann zur Abgabe der Steuersignale aktiviert, wenn nach Vergleich zwischen Grenzwert und Konzentrations-Istwert ein Freigabesignal über eine Freigabeleitung (14) an dem Vergleicher (13) ansteht.

FIG. 1

FIG.2